# EUROPEAN PATENT APPLICATION

(11) **EP 1 374 935 A1**
(43) Date of publication of application: **02.01.2004**
(21) Application number: 02702822.4
(22) Date of filing: 07.03.2002
(51) Int. Cl.: A61M 5/32

(54) **MEDICAL SYRINGE, AND METHOD OF PRODUCING THE SAME**

(30) Priority: 28.03.2001 JP 2001093537
(71) Applicant: Kamata, Miyako, Saitama-shi, Saitama 330-0861 (JP); Miura, Sumiko, Kuroiso-shi, Tochigi 325-0074 (JP)
(72) Inventor: Kamata, Miyako, Saitama-shi, Saitama 330-0861 (JP); Miura, Sumiko, Kuroiso-shi, Tochigi 325-0074 (JP)
(74) Representative: Weise, Reinhard, Dipl.-Ing.
(86) International application number: PCT/JP2002/002161
(87) International publication number: WO 2002/078772

(57) **Abstract**

In an injection needle 1 with a first cut surface 3 formed by simply cutting a cylinder bias and second and third cutting surfaces 4 and 5 provided by cutting the first cut surface 3 so as to have a chevron shape which slopes down toward the outside-diameter side in a bilateral cutting width direction, by further reducing an insertion width, not only skin damage during insertion but also insertion pressure are reduced so that an insertion pain is eliminated and early recovery from the injection mark is realized. The outer circumferential surface of the cylinder of the inj ection needle with the first, second, and third cut surfaces 3, 4, and 5 formed is provided as a curved surface 2 curved in the around-the-needle-axis direction and axial center direction so as to form an arc-shaped outer circumferential edge without a sharp corner part, whereby damage to the inner wall of a blood vessel is avoided during insertion.

## Description

### Technical Field

The present invention belongs to the technical field of medical injection needles (hollow needles and piercing needles) to be employed in overall medical treatment, such as injection of drug solutions, blood drawing, etc.

### Background Art

Nowadays, an injection treatment for directly injecting a drug solution into a body and for obtaining body fluid such as blood and other purposes is widely performed in medical treatment, and for such purposes, an injection needle (a hollow needle) to be inserted into a body via skin is required. In such an inj ection needle, a reduction in pain during insertion, a reduction in damage to human body tissue such as skin and blood vessels (minimization and early recovery etc. of injection needle marks) and the like are strongly demanded.

A needle tip of a conventional needle is formed by simply cutting a cylindrical needle body into a bias (tapered) shape. However, with this inj ection needle, a tissue such as skin which has been widely two-dimensionally cut in a diametrical direction (a cutting width direction) by the needle tip of the injection needle is three-dimensionally pushed and stretched when the needle tip is inserted, therefore, this results in not only a wide insertion width but also a high insertion pressure, thereby not only causing a pain to a person receiving the insertion but also causing a problem such that it takes an extended amount of time to recover from the injection mark and damage to the tissue such as skin and blood vessels is great.

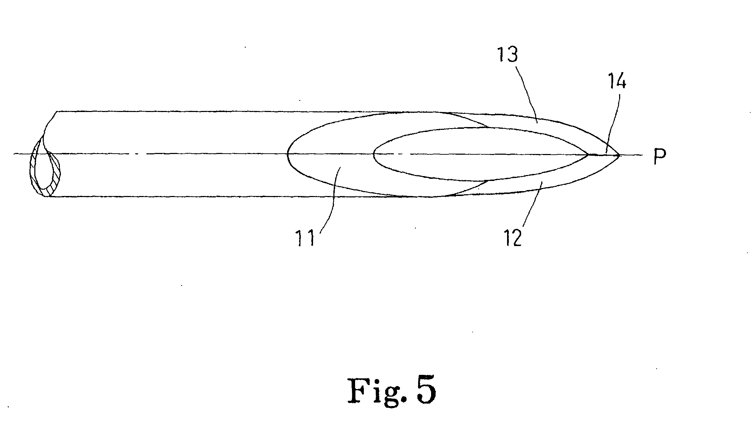

Therefore, concerning tip cutting of an inj ection needle, various proposals have already been made. Human body tissues including skin are incredibly flexible in elasticity, and, accordingly, as one of such proposals, as in a prior art shown in Fig. 5, in addition to a simple bias cut (first cut) surface 11 of the above-described cylinder, at its front end portion, second and third cut surfaces 12 , 13 were provided by cutting the above-described first cut surface 11 so as to have a chevron shape which sloped down toward the outside-diameter side in a diametrical direction (a bilateral cutting width direction) with respect to an axial core line P of the injection needle so that a needle tip with three-dimensional cut surfaces 11, 12, and 13 was provided. Thereby, an insertion into a tissue was made three-dimensional, whereby compared to a conventional two-dimensionally inserting needle tip, the insertion length (insertion width) was reduced to relieve not only skin damage at the time of insertion but also insertion pressure so that an insertion pain was eliminated and an early recovery from the thrust needle mark was realized, and this has been appreciated to a certain extent.

Nevertheless, although injection needles with the above-described second and third cut surfaces 12, 13 have been appreciated to a certain extent and employed for general purposes, needles to inject and eject a large quantity of fluid such as a body fluid or a drug, such as needles for dialysis and for internal placement and blood drawing needles, have a large needle diameter. In such needles, although the conventional problem can be solved to a certain extent due to the presence of said second and third cut surfaces 12 and 13, a ridgeline portion 14 becomes long, and this cuts skin, long in the longitudinal direction at the time of insertion, therein reduction in pain at the time of insertion and insertion pressure and improvement in an early recovery from an insertion wound inevitably have their own limits.

In view of the above, as disclosed in JP-A-H09-56815 (Fig. 6 and Fig. 7) , a needle has been proposed, wherein, in its needle tip portion, a flat fourth cut surface 15 or a bevel cut surface (a conical surface or an umbrella-shaped chamfered surface) 16 is formed on the outer circumferential surface of a side opposite to a side where first, second, and third cut surfaces 11, 12, 13 are formed. Namely, this needle has a flat cut surface 15 of a flat surface-like fourth cut surface or a cone-shaped fourth bevel cut surface 16 formed on the above-described opposite-side outer circumferential surface, whereby compared to the needle with only the second and third tapered surfaces 12 , 13 formed in the aforementioned prior art, the length of the ridgeline 14 is shortened and the needle tip is displaced to the inner-radial side with respect to the outer circumferential surface of the needle so as to reduce damage to skin so that reduction in pain at the time of insertion can be realized.

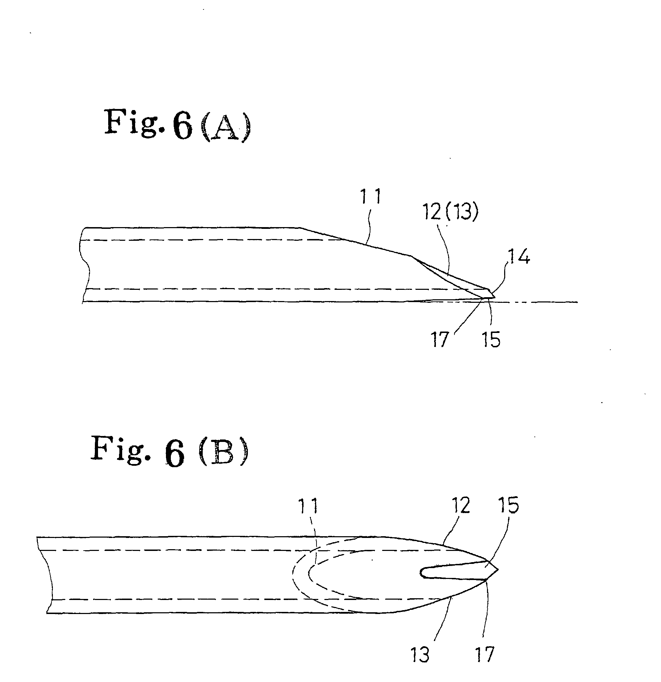

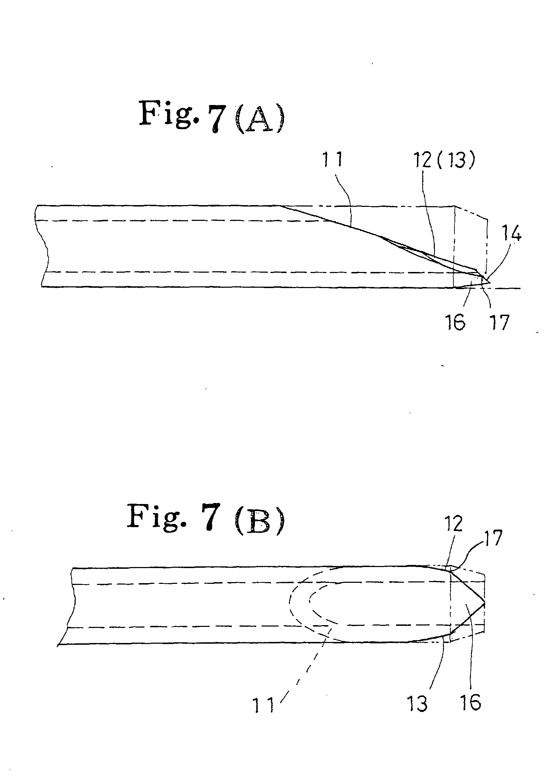

However, since these fourth cut surfaces 15, 16 have a flat surface shape or a conical shape with respect to a needle axial center P direction, as shown in Figs. 6 and 7, an intersection 17 between the second and third cut surfaces 12 , 13 and the fourth cut surface 15/16 results in a sharp corner shape. When such a needle is inserted into a blood vessel, the inserted sharp intersection 17 part exists inside the blood vessel, therefore, if, for example, a patient moves his/her arm and the needle is moved, the intersection 17 part damages the internal surface of the blood vessel, thus resulting in a poor recovery from an injection mark, and moreover, it is presumed that a body tissue such as a blood vessel tissue cut off due to damage flows inside the blood vessel and causes a secondary disorder such as a vascular occlusion, therein exists a problem to be solved by the present invention.

### Disclosure of the Invention

The present invention has been made in light of the above-described circumstances with the aim of solving the problems, and provides a medical injection needle of a hollow shape to be inserted into a body tissue of a human body such as skin, in which, in a tip portion of the injection needle, a first cut surface ground into a bias shape with respect to the axial center direction and second and third cut surfaces where the right and left of the first cut surface are ground into a chevron shape on the tip side of the first cut surface are formed so as to form a ridgeline at the needle tip, wherein the cylinder outer circumferential surface is, in order to shorten the length of the ridgeline, a curved surface which is curved in the around-the-needle-axis direction and axial center direction so that the thickness is reduced toward the needle front side.

With this structure, the length of the ridge line 6 formed by the first and the second cut surfaces can be shortened which will reduce a pain at the time of insertion and lower a insertion pressure during the insertion and at the same time the occurrence of a secondary disorder caused by an injection can be reduced because a human body tissue is prevented from being damaged during the insertion due to non-existence of a sharp corner part.

Therein, the curved surface can be formed from the needle tip up to a position of the first cut surface or can be formed from the needle tip up to a position beyond the first cut surface, whereby tissue damage can be further avoided.

Moreover, the present invention provides a manufacturing method of a medical injection needle of a hollow cylinder shape to be inserted into a body tissue of a human body such as skin, comprising the steps of: forming, on the outer circumferential surface of a tip portion of the injection needle, a curved surface to be curved in the around-the-needle-axis direction and axial center direction so as to reduce the thickness of the cylinder toward the needle tip; forming a first cut surface ground into a bias shape with respect to the axial center direction; and forming second and third cut surfaces where the right and left of the first cut surface are ground in a chevron shape on the tip side to form a ridgeline at the needle tip.

### Brief Description of Drawings

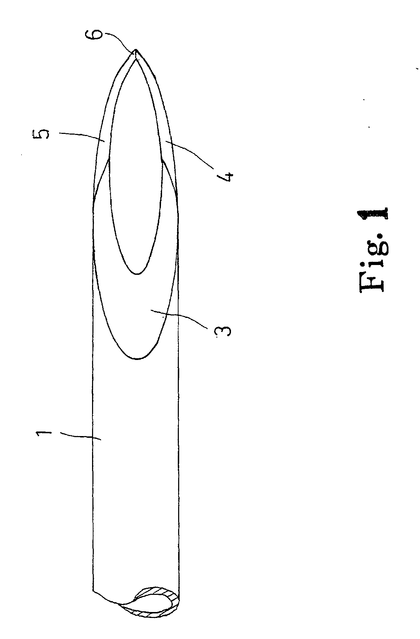

Fig. 1 is an enlarged plan view of a tip portion of an injection needle.

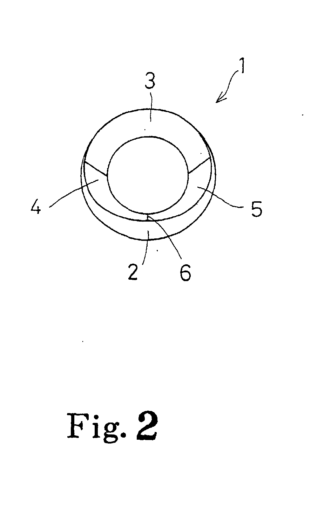

Fig. 2 is an enlarged side view of a tip portion of an injection needle.

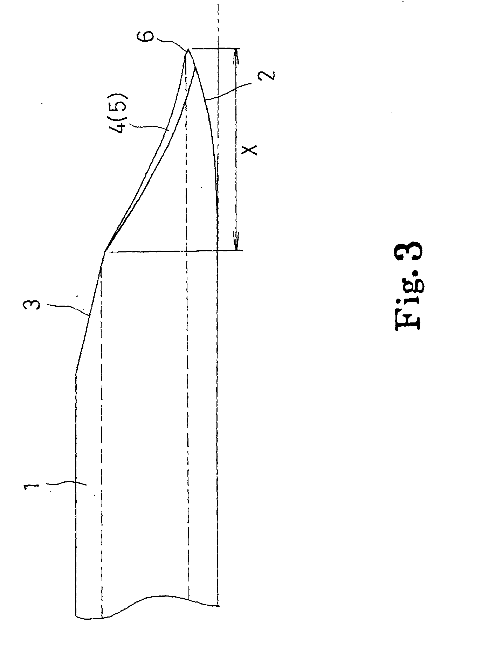

Fig. 3 is an enlarged front view of a tip portion of an injection needle.
Figs. 4 shows an inj ection needle of the second embodiment, wherein (A) is a plan view, (B) is a side view, (C) is a bottom view, and (D) is a side view.
Fig. 5 is an enlarged plan view of a tip portion of an injection needle showing a prior art.
Figs. 6 (A) and (B) are an enlarged front view and an enlarged rear view of a tip portion of an injection needle showing a prior art, respectively.
Figs. 7 (A) and (B) are an enlarged front view and an enlarged rear view of a tip portion of an injection needle showing a prior art, respectively.

### Best Mode for carrying out the Invention

Now, an embodiment of the present invention will be described based on the drawings. In the drawings, 1 denotes an inj ection needle, and in this inj ection needle 1, the present invention has been carried out at a tip portion of its cylindrical (circular tube-like) main needle body so as to ease insertion into a body. That is, at one side of the tip portion of the cylindrical body to form this needle tip portion, the tip is gradually curved in the axial center direction as well as the circumferential direction toward the center side, whereby a curved surface 2 is first formed so that the cylinder thickness is reduced toward the needle tip side. This curved surface 2 is formed by grinding (polishing) the needle tip portion with an elastic grinding stone (corresponding to a step of forming a curved surface) . And, in terms of the tip portion of the main needle body 2, in which the curved surface 2 has been formed on one-side surface of the tip portion as such, a tapered, first cut surface 3 is provided by grounding on an opposite-side surface to the side with the curved surface 2 formed (corresponding to a step of forming a first cut surface) , and on the right and left of its tip side, second and third cut surfaces 4, 5 having a relationship to mutually create a chevron shape are formed (corresponding to a step of forming second and third cut surface) , and at the tip, a chevron shaped ridgeline 6 is formed by these cut surfaces 4, 5 . Herein, since the rear-surface side of the section of the above-described second and third cut surfaces 4, 5 has been formed as the above-described curved surface 2 (the X-range shows the curved surface), a setting has been provided so that the length of the ridgeline 6 becomes short and an outer circumferential edge part has an arc shape without a sharp corner part so as to become an arc-shaped outer circumferential edge as if it were simply formed by grinding to be the first cut surface 3.

In the present embodiment constructed as described, in the injection needle 1, the second and third cut surfaces 4, 5 are formed so that the insertion width with respect to the bilateral width direction (radial direction) can be reduced and moreover, the length of the ridgeline 6 is shortened so that a burden to a person receiving insertion can be reduced, while for a length direction of the ridge line 6, employed is a structure having the curved surface 2, which is not simply linear in the needle axis line direction as in the prior art but is curved also in the axis line direction so as to shorten the length of the ridgeline 6. Therefore, unlike the prior needle whose fourth cut surface was formed in a flat shape or a cone shape to reduce the ridgeline 6, no sharp corner part is formed between the fourth cut surface and second and third cut surfaces 4, 5, and although the needle tip portion has second and third cut surfaces 4 , 5 and the length of the ridge line 6 is shortened, an arc-shaped outer circumferential edge without a sharp corner part is provided. Consequently, when the injection needle 1 is inserted into a human body tissue such as a blood vessel, a problem such that damage to the inner wall of the blood vessel can be reduced, thus resulting in a satisfactory recovery from the injection mark, and moreover, a cutoff of a blood vessel tissue is eliminated to avoid causing a secondary disorder such as a vascular occlusion.

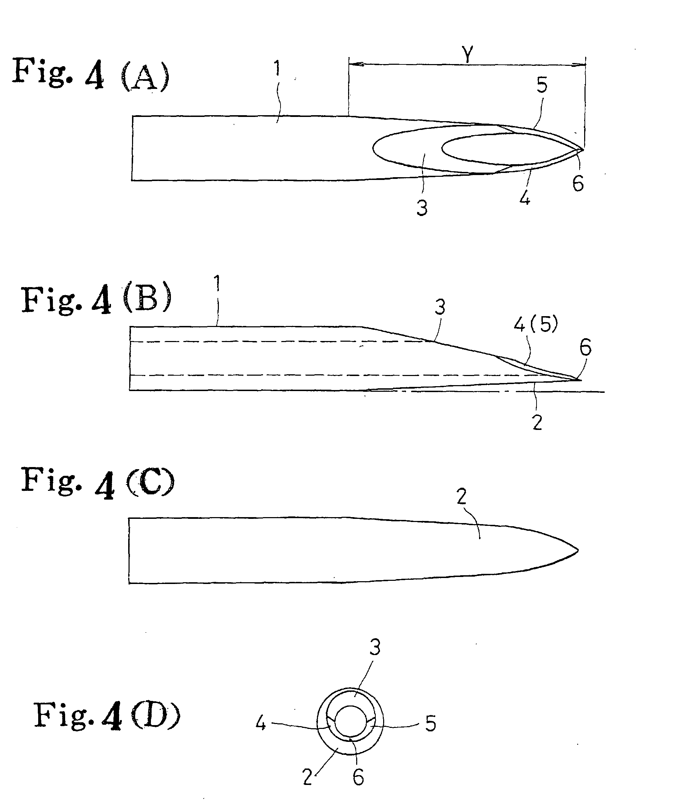

In addition, the present invention is not limited to the above-described embodiment and can be carried out as in a second embodiment as shown in Figs. 4. In this embodiment, a curved surface 2 to be formed on a needle 1 is formed on the entire circumference from the needle tip up to a position beyond a first cut surface 3 (the Y-range shows the curved surface), and then the first cut surface 3 and second and third cut surfaces 4, 5 are formed, and the present invention can be carried out in such a manner as well. In this case, since the thickness from the needle tip as long as a part beyond the first cut surface is reduced, damage to skin is reduced by that extent, therefore, reduction in pain at the time of insertion and improvement in an early recovery can be further realized.

### Industrial Applicability

In brief, since the present invention has been constructed as described above, the length of the ridgeline formed by the second and third cut surfaces is made small so that insertion pressure can be decreased and pain at the time of insertion can be relieved, while presence of a sharp corner part is eliminated to avoid tissue damage in an inserted state so that occurrence of a secondary disorder caused by an inj ection can be reduced, therefore, the present invention has applicability in the industrial field of such medical injection needles.

## Claims

1. A medical injection needle of a hollow shape to be inserted into a body tissue of a human body such as skin, in which, in a tip portion of said injection needle, a first cut surface ground into a bias shape with respect to the axial center direction and second and third cut surfaces where the right and left of said first cut surface are ground into a chevron shape on the tip side of the first cut surface are formed so as to form a ridgeline at the needle tip, wherein
said cylinder outer circumferential surface is, in order to shorten the length of said ridgeline, a curved surface which is curved in the around-the-needle-axis direction and axial center direction so that the thickness is reduced toward the needle front side.

2. The medical injection needle as set forth in claim 1, wherein
the curved surface is formed from the needle tip up to a position of the first cut surface.

3. The medical injection needle as set forth in claim 1, wherein
the curved surface is formed from the needle tip up to a position beyond the first cut surface.

4. A manufacturing method of a medical injection needle of a hollow cylinder shape to be inserted into a body tissue of a human body such as skin, comprising the steps of:
forming, on the outer circumferential surface of a tip portion of said injection needle, a curved surface to be curved in the around-the-needle-axis direction and axial center direction so as to reduce the thickness of the cylinder toward the needle tip;
forming a first cut surface ground into a bias shape with respect to the axial center direction; and
forming second and third cut surfaces where the right and left of said first cut surface are ground in a chevron shape on the tip side to form a ridgeline at the needle tip.
